# EUROPÄISCHE PATENTSCHRIFT

(11) **EP 1 364 678 B1**
(45) Veröffentlichungstag und Bekanntmachung des Hinweises auf die Patenterteilung: **03.12.2008**
(21) Anmeldenummer: 03090211.8
(22) Anmeldetag: 17.12.1997
(51) Int. Cl.: A61N 1/05

(54) **Stimulationselektrodenanordnung**
Lead system
Système d'électrodes de stimulation

(30) Priorität: 17.12.1996 DE 19654491
(43) Veröffentlichungstag der Anmeldung: 26.11.2003
(62) Teilanmeldung aus: 97954335.2
(73) Patentinhaber: BIOTRONIK GmbH & Co. KG, 12359 Berlin (DE)
(72) Erfinder: Thong, Tran, Portland, Oregon 97229 (US); Schaldach, Max, 91054 Erlangen (DE)
(74) Vertreter: Eisenführ, Speiser & Partner

(56) Entgegenhaltungen:
- US-A- 5 571 163
- US-A- 5 662 698

## Beschreibung

Die Erfindung betrifft eine Stimulationselektrodenanordnung für einen Herzschrittmacher.

Bei der Therapie verschiedener chronischer Herzrhythmusstörungen sind seit langem implantierte Herzschrittmacher in Verbindung mit auf einem intrakardialen Elektrodenkatheter angeordneten und an der Herzinnenwand positionierten Stimulationselektroden in Gebrauch, über die das reizbare Herzgewebe erregt und dadurch ein Defekt des körpereigenen kardialen Reizbildungs- und -leitungssystems ausgeglichen wird.

Die Ausgestaltung der Schrittmacher und der zugehörigen Elektrodenleitungen hat zunehmende Vervollkommnung erfahren. Mit dem Ziel einer langfristigen Gewährleistung eines guten Kontaktes zwischen der oder den Stimulationselektrode(n) und dem Herzgewebe im Interesse einer zugleich energiesparenden und sicheren Stimulation sind zahlreiche technische Lösungen zur Verankerung der Elektrodenleitungen an der Herzwand - sowohl in der Herzkammer (Ventrikel) als auch im Vorhof (Atrium) - gefunden worden und tatsächlich wesentliche praktische Verbesserungen gelungen.

Dennoch zählen auch heute noch Elektrodendislokationen, die zu einer Verschlechterung oder einem Verlust des Kontakts mit dem reizbaren Gewebe an der Herzinnenwand und in der Folge zumindest zu einer wesentlichen Erhöhung der Reizschwelle und damit einem erhöhten Stromverbrauch des Schrittmachers und einer verringerten Lebensdauer und schlimmstenfalls zu dessen Funktionsunfähigkeit führen, zu den wichtigsten Komplikationen bei der Schrittmachertherapie. Die technische Weiterentwicklung von (immer komplizierter und aufwendiger werdenden) Verankerungsmechanismen dauert daher noch immer an.

Neben dem hohen Herstellungsaufwand ist bei diesen herkömmlichen Elektrodenanordnungen auch die recht aufwendige und große Erfahrung erfordernde Implantation von Nachteil.

Von vornherein nicht fest in der Herzkammer oder dem Vorhof zu verankernde (sogenannte "flottierende") und daher einfachere, kostengünstigere und leichter zu implantierende Elektrodenanordnungen sind - vorrangig aus implantierbaren Defibrillatoranordnungen, aber auch aus dem Einsatz mit Herzschrittmachern - ebenfalls bekannt.

Eine frühe Anordnung dieser Art zeigt die US-Patentschrift 3 915 174.

In der europäischen Patentanmeldung EP 0 601 338 A1 ist ein Elektrodensystem für einen implantierten Defibrillator beschrieben, das mindestens zwei - ohne besondere Verankerung allein aufgrund ihrer Größe ortsfest gehaltene - intravaskular plazierte Spulenelektroden (spiralförmige Elektroden) umfaßt. Eine dieser großflächigen Defibrillationselektroden kann speziell in der Vena cava superior angeordnet sein.

In der europäischen Patentanmeldung EP-0 677 301-A1 ist ein Elektrodenkatheter zum Einsatz mit einem Defibrillator beschireben, bei dem eine langgestreckte zylindrische Elektrode mit einer längs der Leitung verschiebbaren Abdeckung eine Verschiebung des wirksamen Elektrodenbereiches ermöglicht. Der Katheter kann beispielsweise so eingestellt sein, daß eine erste Elektrode im Ventrikel und der durch Verschiebung der Abdeckung positionierte Elektrodenbereich in der Vena cava superior positioniert ist.

Im Herzen flottierende Stimulationselektroden konnten bei klinischen Untersuchungen in der Vergangenheit nicht mit so hinreichender Sicherheit eine zuverlässige Stimulation mit den verfügbaren Reizimpulsspannung und unter sparsamer Nutzung der begrenzten Batteriekapazität gewährleisten, daß eine nennenswerte praktische Anwendung hätte erfolgen können.

Das herkömmliche Vorgehen, die Stimulationsimpulse eines implantierten Schrittmachers über wandständige Elektroden an das reizbare Muskelgewebe der Herzinnenwandung im unteren Bereich des Ventrikels bzw. des Atriums zu übertragen, kann - ganz abgesehen von den angesprochenen praktischen Problemen - bei einer Reihe häufiger Herzrhythmusstörungen aus grundsätzlichen physiologischen Erwägungen nicht befriedigen: Mit diesem Vorgehen werden nämlich Bereiche auf den unteren hierarchischen Ebenen des kardialen Reizbildungs- und -leitungsystems stimuliert. Dies kann beispielsweise eine technisch aufwendige Zweikammerstimulation (im Atrium und Ventrikel) auch bei Krankheitsbildern erforderlich machen, für die ein Sinusknotendefekt prägend, bei denen das übrige Reizleitungssystem jedoch weitgehend intakt ist.

Die DE-Patentanmeldung 196 09 471.2 der Anmelderin betrifft eine Anordnung mit vorzugsweise flottierenden Stimulationselektroden unterschiedlicher Polarität zur Erzeugung eines auf vorbestimmte Herzgewebsbereiche "fokussierten" Reizimpulsfeldes.

In der US-A-5,235,977 ist eine Elektrodenanordnung für Defibrillierungszwecke angegeben, welche zwei intravaskuläre Elektroden und eine Flächenelektrode außerhalb des Herzens aufweist. Die intravaskulären Elektroden sind in der Vena Cava Superior angeordnet.

Die US-A-5,314,430 offenbart einen atriellen Defibrillationspulsgenerator mit einem Elektrodenableitsystem. Das Elektrodenableitsystem umfasst eine Koronarsinus-Elektrode und eine Subkutan-Elektrode, welche vorzugsweise auf dem Gehäuse des implantierbaren Defibrillators angebracht ist. Das Elektrodensystem kann auch zusätzliche Elektroden zur Anbringung im rechten Ventrikel und/oder in der Vena Cava Superior umfassen.

Eine weitere Elektrodenanordnung für Defibrillierungszwecke ist aus der US-A-5,571,163 bekannt, diese offenbart eine Elecktrodenleitung mit vier Elektroden, von denen eine in der Vena Cava Superior positioniert werden kann, zwei weitere im implantierten Zustand im rechten Vorhof positioniert werden, und am distalen Ende der Elektrodenleitung ebenfalls eine Elektrode vorhanden ist.

Der Erfindung liegt die Aufgabe zugrunde, eine verbesserte Elektrodenanordnung der eingangs genannten Gattung anzugeben, die einen hinsichtlich der Reizimpulslokalisierung besser den physiologischen Gegebenheiten folgenden und zuverlässigeren Betrieb eines Herzschrittmachers ermöglicht sowie einfach herzustellen und leicht und sicher zu positionieren ist.

Diese Aufgabe wird durch eine Stimulationselektrodenanordnung mit den Merkmalen des Anspruchs 1 gelöst.

Die Erfindung schließt den Gedanken ein, eine Elektrodenanordnung zu realisieren, mit der die Stimulationsimpulse des Schrittmachers den in der Reizbildungshierarchie hochstehenden Bereichen des kardialen Reizbildungs- und -leitungssystems - primär dem Sinusknoten - zugeführt werden können, um etwa einen Sinusknotendefekt auf möglichst einfache Weise mittels präzise lokalisierter elektrischer Stimulation am Ort des Defekts ausgleichen zu können.

Zur Ausführung dieses Gedankens bedient sich die vorgeschlagene Elektrodenanordnung einer Lokalisierung des aus einer einzelnen Stimulationsspannung erzeugten Reizimpulsfeldes im Sinusknoten bzw. einem sonstigen zentralen Bereich des Reizbildungssytems. Dazu dienen in herznahen Blutgefäßen und wahlweise im Vorhof in unmittelbarer Nachbarschaft dieses Bereiches (bevorzugt verankerungsfrei) angeordnete Stimulationselektroden.

Zumindest die erste Stimulationselektrode im herznahen Blutgefäß ist bevorzugt auf einer zur Erstreckung mindestens bis in den Vorhof und zur verankerungsfreien, insbesondere schwimmenden, Verlegung ausgebildeten Elektrodenleitung angeordnet.

Die zweite Stimulationselektrode ist in einer bevorzugten Fortbildung dieser Ausführung in solchem Abstand von der ersten Stimulationselektrode auf der Elektrodenleitung angeordnet, daß sie im implantierten Zustand im Vorhof positioniert ist.

Die Elektrodenleitung kann weiter eine oder auch mehrere distal von der ersten bzw. zweiten Stimulationselektrode derart beabstandete Elektrode(n) aufweisen, daß diese im implantierten Zustand in der Herzkammer angeordnet ist bzw. sind. Diese Ventrikelelektrode(n) kann/können insbesondere als Abfühlelektrode(n), bei speziellen Herzrhythmusstörungen - etwa vollständigem AV-Block - aber auch als zusätzliche Stimulationselektrode (n) dienen.

Die erste Stimulationselektrode - oder die Elektrodenleitung, falls eine solche vorgesehen ist - kann insbesondere Positionierungsmittel aufweisen, mittels derer eine vorbestimmte Position der ersten Stimulationselektrode in der Vena cava superior und/oder der zweiten Stimulationselektrode im Vorhof einstellbar ist.

Die erwähnten Positionierungsmittel sind in zweckmäßiger Weise als Teil(e) der Elektrodenleitung ausgebildet und umfassen in einer vielfältig einstellbaren Ausführung Mittel zur Einstellung einer vorbestimmten Krümmung der Elektrodenleitung im implantierten Zustand.

Alternativ oder zusätzlich hierzu können die Positionierungsmittel elektrisch isolierende oder von den Stimulationselektroden isolierte Abstandhalter umfassen.

Des weiteren können sie von außerhalb des Körpers betätigbare Mittel zur Veränderung der Position der ersten und/oder zweiten Stimulationselektrode im implantierten Zustand zur nachträglichen Lagekorrektur der Elektroden bezüglich des zu stimulierenden Herzgewebsbereiches aufweisen.

Ihrer Stimulationsfunktion entsprechend, sind die erste und/oder die zweite Stimulationselektrode ring- oder ringsegmentförmig mit einem Länge/Durchmesser-Verhältnis kleiner als 1, ausgebildet. (Unter diese Charakterisierung soll auch eine Ausbildung der ersten oder zweiten Elektrode als annähernd zylinder- bzw. "topf"förmige Spitzenelektrode am distalen Ende einer Zuleitung fallen.)

Weiterhin weisen die erste und/oder zweite Stimulationselektrode bevorzugt eine geometrische Oberfläche von weniger als 10 mm² und insbesondere eine fraktale Oberflächen-Mikrostruktur zur Vergrößerung der elektrisch wirksamen Oberfläche um einen Faktor von mindestens 10² auf.

Bei einer hinsichtlich der erreichbaren Reizimpulsfeldverteilung besonders variablen Ausführung ist als weitere Stimulationselektrode eine außerhalb des Herzens angeordnete indifferente Flächenelektrode vorgesehen, die insbesondere als Teil des Gehäuses des Herzschrittmachers realisiert sein kann.

Die präzise Lokalisierung eines hinreichend großen Potentialgradienten durch die beanspruchte Elektrodenanordnung ermöglicht die Überschreitung der Reizschwelle in durch die Elektroden nicht direkt kontaktierten Gewebsbereichen und damit die Reizung der nicht an der Oberfläche der Herzwand und zudem in Gebieten des Herzens, in denen eine aktive Fixierung von Elektroden praktisch unmöglich ist, liegenden Zielbereiche für die Erregung, nämlich des Sinusknotens oder ggfs. des AV-Knotens oder anderer hierarchisch relativ hochstehender Bereiche des Reizbildungs- und -leitungssystems.

Da eine Fixierung der Elektrodenleitung an der Herzwand bei der vorgeschlagenen Stimulationselektrodenanordnung nicht erforderlich ist, kann diese im Herzen "schwimmen". Sie wird mithin zweckmäßigerweise mit einer solchen Biegesteifigkeit aufgebaut und gegebenenfalls. einer solchen vorgeprägten Krümmung im Längsverlauf versehen, daß die Elektroden nach Einführung im wesentlichen nicht in direktem Kontakt mit der Gefäß- bzw. Vorhofwandung stehen, aber nahe dem zu reizenden Bereich angeordnet sind.

Die Krümmung bzw. das Abstehen von Abstandhaltern von der Elektrodenleitung werden durch geeignete (als solche bekannte) Mittel nach der Einführung erzeugt, bevorzugt etwa durch Elemente aus einer bei Körpertemperatur aktivierten Gedächtnislegierung oder Vorspannelemente, die bei Entfernung eines zum Einführen dienenden Führungsdrahtes aus der Elektrodenleitung eine vorgeprägte Form annehmen. Es ist auch möglich, Mittel zur Veränderung der Krümmung von außen vorzusehen, etwa einen ähnlich wie ein Führungsdraht wirkenden Versteifungsdraht.

Bei einer anderen vorteilhaften Ausführungsform sind unterschiedliche Elektrodenbereiche, welche entweder auf der Elektrodenleitung angeordnet sind oder durch das Schrittmachergehäuse gebildet werden gleichzeitig oder alternativ einschaltbar, so daß eine unterschiedliche Feldverteilung, insbesondere auch durch ferngesteuert-programmierte Umschaltung, erzielbar ist. Auf diese Weise kann unterschiedlichen physiologischen Anforderungen Rechnung getragen werden. Für das Atrium sind trotzdem lediglich zwei (interne bzw. externe Anschlußleitungen des Stimulators erforderlich.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine schematische Darstellung des Reizbildungs- und Erregungsleitungssystems des Herzens,
Figur 2 eine schematische Darstellung einer Stimulationselektrodenanordnung gemäß einer Ausführungsform der Erfindung,
Figur 3 eine schematische Darstellung einer Stimulationselektrodenanordnung gemäß einer zweiten Ausführungsform der Erfindung,
Figur 4 eine schematische Darstellung einer Stimulationselektrodenanordnung gemäß einer dritten Ausführungsform der Erfindung sowie
Figur 5 eine schematische Darstellung einer Stimulationselektrodenanordnung gemäß einer vierten Ausführungsform der Erfindung.

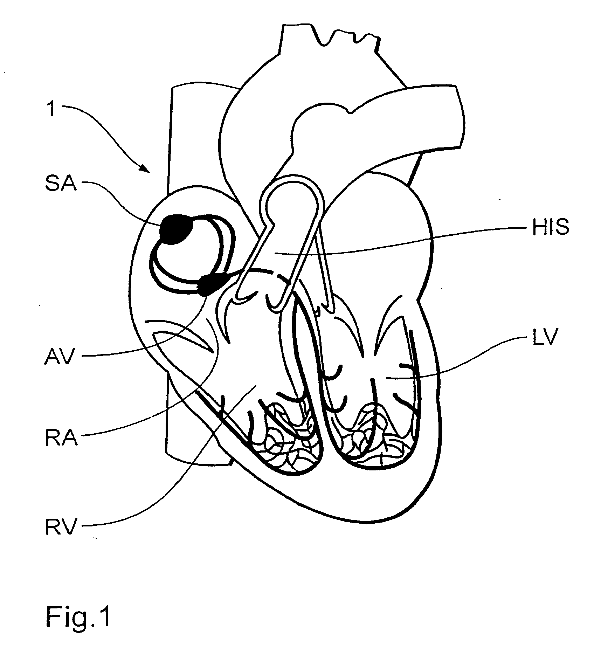

Die Lage der Elemente des Reizbildungs- und Erregungsleitungssystems im Herzen 1, wozu auch das oben nicht erwähnte His-Bündel und der jeweils in die Purkinje-Fasern mündende linke bzw. rechte Schenkel ("Left" bzw. "Right Bundle Branch") gehören, ist zunächst in Fig. 1 skizzenartig gezeigt. Es ist zu ersehen, daß mit der üblichen Stimulation über wandständig verankerte Elektroden in den unteren Regionen der Herzkammer (Ventrikel - LV bzw. RV) und/oder im Vorhof (Atrium - RA) lediglich periphere Bereiche des Systems erregt werden können, so daß auf Defekte in zentraleren Bereichen, etwa dem Sinusknoten (SA) oder AV-Knoten (AV), nicht direkt Einfluß genommen werden kann.

Nachfolgend werden ausgewählte Beispiele für konkrete Stimulationselektrodenanordnungen angegeben, die vom Grundgedanken der Erfindung Gebrauch machen, jedoch nicht im Sinne einer Beschränkung zu verstehen sind. Der Fachmann kann unter Rückgriff auf Details bekannter Elektrodenleitungen ohne weiteres eine Vielzahl weiterer Konfigurationen ausführen, die ebenfalls in den Schutzbereich der Ansprüche fallen.

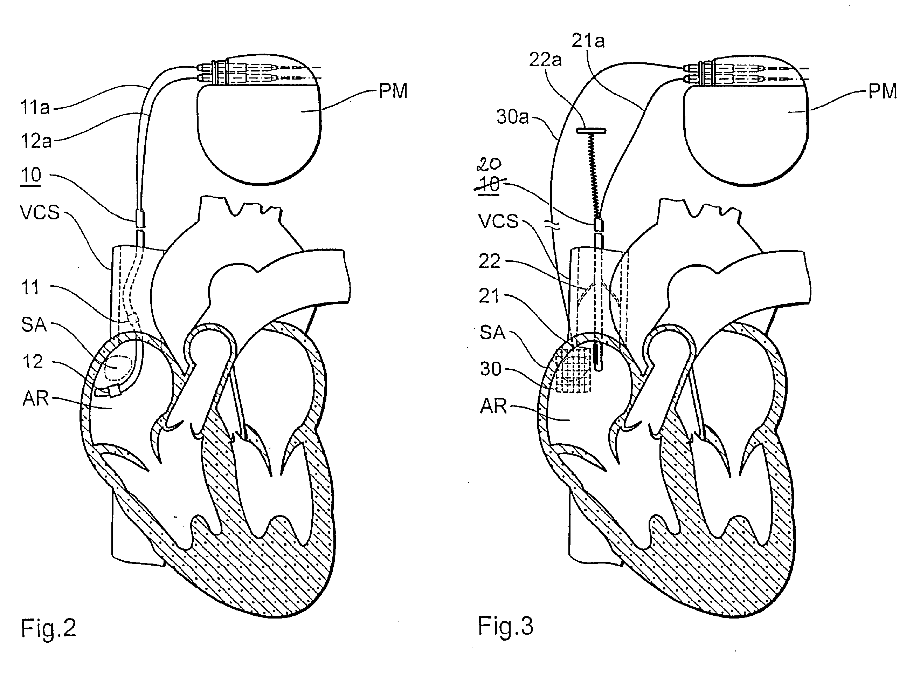

Fig. 2 ist eine schematische Darstellung einer mit S-förmig gekrümmtem Verlauf über die Vena cava superior VCS schwimmend in das rechte Atrium AR des Herzens H verlegte Elektrodenleitung 10. Auf der Leitung 10 sind eine erste, im dargestellten implantierten Zustand in der Vena cava positionierte Stimulationselektrode 11 und mit geringem Abstand zum distalen Leitungsende eine zweite, im hohen Atrium positionierte Stimulationselektrode 12 gebildet. Beide Elektroden 11, 12 sind als Ringelektroden mit (was in der Figur nicht erkennbar ist) fraktalartig mikrostrukturierter Oberfläche ausgeführt. Der mit vorgeprägter Krümmung ausgeführte Leitungskörper wird für die Einführung in bekannnter Weise durch einen (nicht gezeigten) Führungsdraht gestreckt und bei der Implantation in eine solche Winkelstellung gedreht, daß die Elektroden den Sinusknoten SA in möglichst geringem Abstand im wesentlichen zwischen sich einschließen. Die Elektroden 11, 12 sind über Zuleitungen 11a, 12a zu bipolarem Betrieb mit einem Herzschrittmacher PM verbunden und stimulieren bei Ausgabe eines Stimulationsimpulses an den Schrittmacherausgängen direkt den Sinusknoten SA.

Fig. 3 ist eine entsprechende Darstellung einer mir dem distalen Ende bis in den Übergangsbereich zwischen Vena cav superior VCS und rechtem Atrium AR reichenden Elektrodenleitung 20, welche an ihrem distalen Ende eine im Querschnitt halbkreisförmige Elektrode 21 aufweist. Diese ist über eine Zuleitung 21a mit einem Pol eines Herzschrittmachers PM verbunden, an dessen anderen Ausgang eine extrakardial, aber nahe dem Sinusknoten SA implantierte, relativ kleine Flächenelektrode 30 angeschlossen ist. (Je nach patientenspezifischer Lage des Sinusknotens ist ggfs. eine Einführung der zweiten Elektrode in ein herznahes Gefäß möglich und zu bevorzugen, da diese den Operationsaufwand verringert.) Die Position der Leitung 20 und der ersten Elektrode 21 im Gefäß SVC und damit relativ zum Sinusknoten SA wird durch von außen über einen Betätigungsgriff 22a gesteuert abspreizbare Abstandshalter 22 eingestellt. Auch bei dieser Anordnung bildet sich - korrekte Positionierung der Elektroden 21, 30 vorausgesetzt - bei Ausgabe eines Reizimpulses durch den Schrittmacher PM ein den Sinusknoten durchsetzendes Reizimpulsfeld aus.

Unter Fortlassung der extrakardialen Elektrode 30 kann mit der in Fig. 3 gezeigten Elektrodenleitung 20 grundsätzlich auch eine unipolare Stimulation gegen des Gehäuse des Schrittmachers PM als Referenz ausgeführt werden, was - um den Preis eines tendentiell höheren Energieverbrauchs und etwas erhöhter Störanfälligkeit - den technischen und operativen Aufwand verringert. Des weiteren können mit dem Schrittmachergehäuse als Referenzelektrode die bipolaren Anordnungen aus Fig. 2 und 3 zu tripolaren Stimulationsanordnungen ergänzt werden, was eine weiter verfeinerte Steuerung der Feldverteilung ermöglicht.

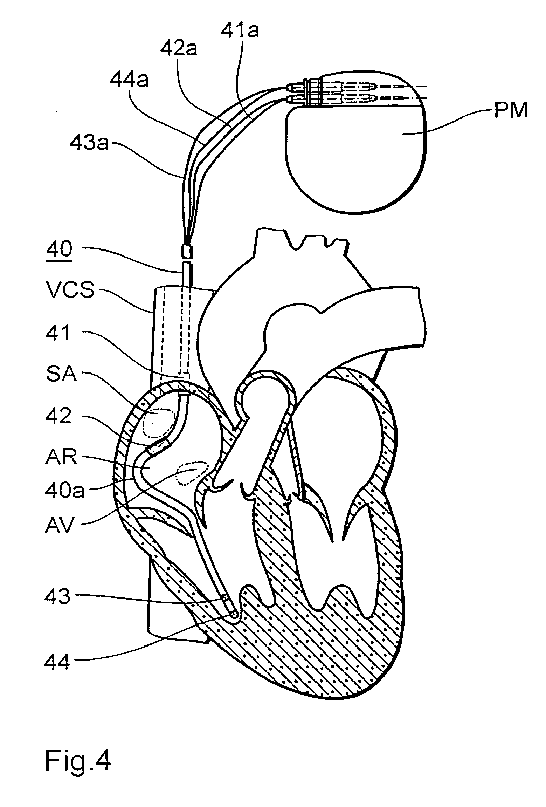

Fig. 4 zeigt eine schematische Darstellung einer dritten Ausführungsform, bei der eine bis in den Apex des rechten Ventrikels VR reichende Elektrodenleitung 40 insgesamt vier Elektroden 41 bis 44 mit entsprechenden Zuleitungen 41a bis 44a aufweist. Das proximale Elektrodenpaar 41, 42, das nach der Implantation der Leitung - wie aus der Figur ersichtlich - in der Vena cava superior bzw. im rechten Atrium liegt, dient wieder zur direkten Stimulation des Sinusknotens SA. Durch eine Ausbiegung 40a der Leitung 40 in vorbestimmtem Abstand vom distalen Ende - verwirklicht etwa in an sich bekannter Weise mit Hilfe von (nicht gezeigten) Leitungsbestandteilen aus einer Gedächtnislegierung - wird eine Stabilisierung der Position der Elektroden 41, 42 ohne regelrechte Verankerung in der Herzwand erreicht.

Die am distalen Leitungsende angeordnete Spitzenelektrode 44 und die von dieser einige Millimeter bis zu etwa 2 cm beabstandete Ringelektrode 43, die nach der Implantation im Ventrikel VR liegen, dienen in an sich bekannter Weise zur bipolaren Erfassung von Kammeraktionen zur Steuerung des Schrittmachers PM und ggfs. auch zur zusätzlichen Stimulation im Ventrikel - etwa bei Patienten mit komplettem AV-Block, bei denen die dem Sinusknoten zugeführten Reizimpulse nicht in den Ventrikel übergeleitet werden.

Es ist ersichtlich, daß der mit der Erfindung beabsichtigte Effekt auch mit Gruppen aus mehreren Elektroden erreicht werden kann. So können auch zwei oder mehr Elektroden im Atrium angeordnet oder aber auch eine weitere Elektrode in der Vena cava superior gelegen sein. Der Einsatz der einen oder der anderen Elektrodenanordnung kann von der tatsächlichen Lage des Sinusknotens bei einem Patienten und von dessen Reizschwelle abhängen.

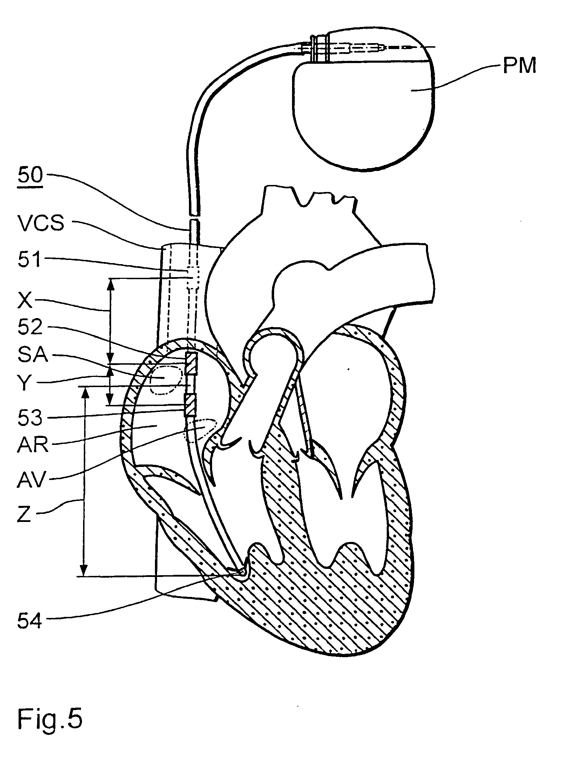

Fig. 5 zeigt eine schematische Darstellung einer derartigen vierten Ausführungsform, bei der eine bis in den Apex des rechten Ventrikels VR reichende Elektrodenleitung 50 insgesamt vier Elektroden 51 bis 54 mit einer Zuleitung 50 aufweist - wobei die einzelnen Leitungsadern für die einzelnen Elektroden hier nicht näher dargestellt sind, da sie ohnehin im Inneren der gemeinsamen Leitung 50 vorgesehen sind. Das proximale Elektrodenpaar 51, 52 bzw. 53, das nach der Implantation der Leitung - wie aus der Figur ersichtlich - in der Vena cava superior bzw. im rechten Atrium liegt, dient wieder zur direkten Stimulation des Sinusknotens SA. Dabei sind die bei bipolarer Stimulation die Elektroden 52 und 53 alternativ als Gegenelektrode für 51 einsetzbar, wobei zusätzlich auch das Schrittmachergehäuse PM in die Stimulation einbezogen werden kann. Die mit Befestigungsmitteln in Form von Tines versehene Ventrikelelektrode 54 ist unipolar mit dem Schrittmachergehäuse PM als Gegenelektrode angeschlossen.

Verschiedene Möglichkeiten der Verschaltung sind in der nachfolgenden Tabelle dargestellt, wobei in den Zeilen mit A1 und A2 und V1 und V2 jeweils die beiden Anschlüsse des Schrittmachers für Atrium und Ventrikelstimulation bzw. - sense bezeichnet sind. Dabei ist im dargestellten Beispiel die Ventrikelelektrode konstant als unipolare Elektrode angeschlossen, während die im Bereich des Atriums verlegten Ringelektroden 51 und 52 alternativ bipolar zusammen mit der Vena-Cava-Elektrode betreibbar sind (Spalten A und B der Tabelle). In C ist eine der Ringelektroden 51 bis 53 unipolar gegen das Schrittmachergehäuse PM geschaltet. In D ist eine der Elektroden 51 bis 53 gegen die weitere Elektrode 52, welche ihrerseits mit dem Schrittmachergehäuse PM verbunden ist geschaltet. In Spalte E sind zwei der Elektroden 51 bis 53 gemeinsam gegen das Schrittmachergehäuse geschaltet. In Spalte F sind zwei der Elektroden 51 bis 53 gemeinsam gegen das Schrittmachergehäuse geschaltet, welches seinerseits mit einer der Elektroden dieser Gruppe zusammengeschaltet ist.

Der Abstand X der beiden Elektroden 51 (vena cava) und 52 (Atrium) beträgt vorteilhafterweise ca. 15 bis 20 mm, wobei die beiden Atriumelektroden 52 und 53 unter sich ca. 10 mm voneinander entfernt sind (Abstand Y). Der Abstand Z zwischen den Atriumelektroden 52 und 53 und der Ventrikelelektrode 54 beträgt ca. 120 bis 140 mm.

Es ist ersichtlich, daß auf diese Weise eine Vielzahl von Verschaltungsmöglichkeiten für die Elektroden im Atriumbereich gegeben ist, denen gemeinsam ist, daß durch die Überlagerung der durch mehrere Elektroden erzeugten Feldverteilung eine unterschiedlichen Erfordernissen entsprechende Stimulationswirkung erzeugt werden kann, wobei sich eine Konzentration der Feldlinien jeweils in besonders auswählbaren Bereichen ergibt. Dabei können alle Spannungen vom Atriumanschluss abgeleitet werden. In den Fällen der Spalten C und E braucht der Schrittmacheranschluß auch für das Atrium nur unipolar zu sein.

| | ***A*** | ***B*** | ***C*** | ***D*** | ***E*** | ***F*** |
|---|---|---|---|---|---|---|
| ***V1*** | PM | PM | PM | PM | PM | PM |
| ***V2*** | 54 | 54 | 54 | 54 | 54 | 54 |
| ***A1*** | 51 | 51 | PM | 52,PM | 51,52 | 51,52 |
| ***A2*** | 53 | 52 | 53 | 51 | PM | PM,53 |

Die am distalen Leitungsende angeordnete Spitzenelektrode 54 und die von dieser wenige Millimeter bis zu etwa 2 cm beabstandete Ringelektrode 53, die nach der Implantation im Ventrikel VR liegen, dienen in an sich bekannter Weise zur bipolaren Erfassung von Kammeraktionen zur Steuerung des Schrittmachers PM und ggfs. auch zur zusätzlichen Stimulation im Ventrikel - etwa bei Patienten mit komplettem AV-Block, bei denen die dem Sinusknoten zugeführten Reizimpulse nicht in den Ventrikel übergeleitet werden.

Mit ganz ähnlichen Anordnungen wie den oben beschriebenen ist auch eine Reizimpulsfeldlokalisierung im AV-Knoten (vgl. Fig. 1) oder einem anderen zentralen Bereich des Reizbildungs- und -leitungssystems erreichbar.

## Patentansprüche

1. Stimulationselektrodenanordnung zur Stimulation des Herzens (H) mittels eines implantierbaren Herzschrittmachers (PM),
mit einer im implantierten Zustand bis in das Apex des rechten Ventrikels reichenden Elektrodenleitung (50) mit vier auf der Elektrodenleitung gebildeten Stimulationselektroden (51, 52,53, 54) mit einer Zuleitung, wobei die Stimulationselektroden auf der Elektrodenleitung derart angeordnet sind, dass im implantierten Zustand
eine erste Stimulationselektrode (51) in der Vena Cava Superior (VCS) positioniert ist,
eine zweite Stimulationselektrode (52) und eine dritte Stimulationselektrode (53) im implantierten Zustand im rechten Vorhof (AR) positioniert sind, und
eine vierte Stimulationselektrode (54) am distalen Ende der Elektrodenleitung angeordnet ist und unipolar mit dem Schrittmachergehäuse (PM) als Gegenelektrode angeschlossen ist
**dadurch gekennzeichnet, dass**
die erste Stimulationselektrode ring- oder ringsegmentförmig mit einem Länge/Durchmesser-Verhältnis von kleiner als 1 ausgebildet ist und eine geometrische Oberfläche von weniger als 10 mm² aufweist.

2. Stimulationselektrodenanordnung nach Anspruch 1, **dadurch gekennzeichnet, dass** die zweite Stimulationselektrode (52) und die dritte Elektrode (53) alternativ mit der ersten Stimulationselektrode (51) als Gegenelektrode bipolar betreibbar sind.

3. Stimulationselektrodenanordnung nach Anspruch 1, **dadurch gekennzeichnet, dass**
die erste Stimulationselektrode (51), die zweite Stimulationselektrode (52) oder die dritte Stimulationselektrode (53) unipolar gegen das Schrittmachergehäuse (PM) schaltbar ist.

4. Stimulationselektrodenanordnung nach Anspruch 1, **dadurch gekennzeichnet, dass** die erste Stimulationselektrode (51) oder die dritte Stimulationselektrode (53) gegen die zweite Stimulationselektrode (52) schaltbar ist, wobei die zweite Stimulationselektrode (52) mit dem Schrittmachergehäuse (PM) verbunden ist.

5. Stimulationselektrodenanordnung nach Anspruch 1, **dadurch gekennzeichnet, dass** zwei der Stimulationselektroden
erste Stimulationselektrode (51), zweite Stimulationselektrode (52) und dritte Stimulationselektrode (53)
gemeinsam gegen das Schrittmachergehäuse (PM) schaltbar sind.

6. Stimulationselektrodenanordnung nach Anspruch 1, **dadurch gekennzeichnet, dass** zwei der Stimulationselektroden aus einer Gruppe
erste Stimulationselektrode (51), zweite Stimulationselektrode (52) und dritte Stimulationselektrode (53)
gemeinsam gegen das Schrittmachergehäuse (PM) schaltbar sind, wobei das Schrittmachergehäuse (PM) mit einer der Stimulationselektroden der Gruppe zusammenschaltbar ist.

7. Stimulationselektrodenanordnung nach einem der vorhergehenden Ansprüche, **dadurch gekennzeichnet, dass** der Abstand (X) der ersten Stimulationselektrode (51) und der zweiten Stimulationselektrode (52) cirka 15 bis 20 mm beträgt, wobei die zweite Stimulationselektrode (52) und dritte Stimulationselektrode (53)als Atriumelektroden unter sich cirka 10 mm voneinander entfernt sind (Y), und der Abstand (Z) zwischen den Atriumelektroden (52, 53) und der vierten Stimulationselektrode (54) cirka 120 bis 140 mm beträgt.

## Claims

1. A stimulation electrode arrangement for stimulation of the heart (H) by means of an implantable heart pacemaker (PM),
having an electrode line (50) that extends into the apex of the right ventricle in the implanted state and has four stimulation electrodes (51, 52, 53, 54) formed on the electrode line having a feeder line, such that the stimulation electrodes are arranged on the electrode line in such a way that in the implanted state,
a first stimulation electrode (51) is positioned in the superior vena cava (SVC),
a second stimulation electrode (52) and a third stimulation electrode (53) are positioned in the right atrium (AR) in the implanted state, and
a fourth stimulation electrode (54) is arranged on the distal end of the electrode line and is connected in unipolar fashion to the pacemaker housing (PM) as a counterelectrode,
**characterized in that**
the first stimulation electrode is designed in the form of a ring or ring segment with a length/diameter ratio of less than 1 and a geometric surface area of less than 10 mm².

2. The stimulation electrode arrangement according to Claim 1,
**characterized in that**
the second stimulation electrode (52) and the third electrode (53) can alternatively be operated as bipolar electrodes with the first stimulation electrode (51) as a counterelectrode.

3. The stimulation electrode arrangement according to Claim 1,
**characterized in that**
the first stimulation electrode (51), the second stimulation electrode (52) or the third stimulation electrode (53) can be switched to the pacemaker housing (PM) for unipolar operation.

4. The stimulation electrode arrangement according to Claim 1,
**characterized in that**
the first stimulation electrode (51) or the third stimulation electrode (53) can be switched to the second stimulation electrode (52), such that the second stimulation electrode (52) is connected to the pacemaker housing (PM).

5. The stimulation electrode arrangement according to Claim 1,
**characterized in that**
two of the stimulation electrodes,
a first stimulation electrode (51), second stimulation electrode (52) and third stimulation electrode (53)
can be switched jointly to the pacemaker housing (PM).

6. The stimulation electrode arrangement according to Claim 1,
**characterized in that**
two of the stimulation electrodes from the group, comprising
a first stimulation electrode (51), second stimulation electrode (52) and third stimulation electrode (53)
can be switched jointly to the pacemaker housing (PM) such that the pacemaker housing (PM) can be switched together with one of the stimulation electrodes of the group.

7. The stimulation electrode arrangement according to any one of the preceding claims,
**characterized in that**
the distance (X) of the first stimulation electrode (51) and the second stimulation electrode (52) amounts to approximately 15 mm to 20 mm such that the second stimulation electrode (52) and the third stimulation electrode (53) as the atrium electrodes are a distance of approximately 10 mm apart from one another (Y) and the distance (Z) between the atrium electrodes (52, 53) and the fourth stimulation electrode (54) is approximately 120 mm to 140 mm.

## Revendications

1. Agencement d'électrodes de stimulation pour la stimulation du coeur (H) au moyen d'un pacemaker (PM) implantable,
comprenant une ligne d'électrodes (50) allant dans l'état implanté jusque dans l'apex du ventricule droit avec quatre électrodes de stimulation (51, 52, 53, 54) formées sur la ligne d'électrodes avec une arrivée, les électrodes de stimulation étant disposées sur la ligne d'électrodes, de telle sorte que, dans l'état implanté,
une première électrode de stimulation (51) est positionnée dans la veine cave supérieure (VCS),
une seconde électrode de stimulation (52) et une troisième électrode de stimulation (53) sont positionnées dans l'état implanté dans l'oreillette droite (AR) et
une quatrième électrode de stimulation (54) est disposée sur l'extrémité distale de la ligne d'électrodes et est raccordée de façon unipolaire au boîtier de pacemaker (PM) comme contre-électrode,
**caractérisé en ce que**
la première électrode de stimulation est conçue avec une forme annulaire ou une forme de segment annulaire avec un rapport longueur/diamètre inférieur à 1 et présente une surface géométrique de moins de 10 mm².

2. Agencement d'électrodes de stimulation selon la revendication 1, **caractérisé en ce que** la seconde électrode de stimulation (52) et la troisième électrode (53) peuvent être exploitées alternativement avec la première électrode de stimulation (51) comme contre-électrode de façon bipolaire.

3. Agencement d'électrodes de stimulation selon la revendication 1, **caractérisé en ce que**
la première électrode de stimulation (51), la seconde électrode de stimulation (52) ou la troisième électrode de stimulation (53) peut être commutée de façon unipolaire par rapport au boîtier de pacemaker (PM).

4. Agencement d'électrodes de stimulation selon la revendication 1, **caractérisé en ce que** la première électrode de stimulation (51) ou la troisième électrode de stimulation (53) peut être commutée par rapport à la seconde électrode de stimulation (52), la seconde électrode de stimulation (52) étant reliée au boîtier de pacemaker (PM).

5. Agencement d'électrodes de stimulation selon la revendication 1, **caractérisé en ce que** deux des électrodes de stimulation
première électrode de stimulation (51), seconde électrode de stimulation (52) et troisième électrode de stimulation (53),
peuvent être commutées ensemble par rapport au boîtier de pacemaker (PM).

6. Agencement d'électrodes de stimulation selon la revendication 1, **caractérisé en ce que** deux des électrodes de stimulation provenant d'un groupe
première électrode de stimulation (51), seconde électrode de stimulation (52) et troisième électrode de stimulation (53)
peuvent être commutées conjointement par rapport au boîtier de pacemaker (PM), le boîtier de pacemaker (PM) pouvant être commuté conjointement avec l'une des électrodes de stimulation du groupe.

7. Agencement d'électrodes de stimulation selon l'une quelconque des revendications précédentes, **caractérisé en ce que** l'espacement (X) de la première électrode de stimulation (51) et de la seconde électrode de stimulation (52) est d'environ 15 à 20 cm, la seconde électrode de stimulation (52) et la troisième électrode de stimulation (53) en tant qu'électrodes d'oreillette (53) étant éloignées d'environ 10 mm l'une de l'autre (Y), et la distance (Z) entre les électrodes d'oreillette (52, 53) et la quatrième électrode de stimulation (54) étant d'environ 120 à 140 mm.
